Europäisches Patentamt

European Patent Office

Office européen des brevets

(11) Veröffentlichungsnummer: **0 151 411**
**A1**

(12) **EUROPÄISCHE PATENTANMELDUNG**

(21) Anmeldenummer: 85100428.3

(22) Anmeldetag: 17.01.85

(51) Int. Cl.⁴: **A 61 K 31/43**
//(A61K31/43, 31:425)

(30) Priorität: 03.02.84 CH 512/84

(43) Veröffentlichungstag der Anmeldung:
14.08.85 Patentblatt 85/33

(84) Benannte Vertragsstaaten:
AT BE CH DE FR GB IT LI NL

(71) Anmelder: F. HOFFMANN-LA ROCHE & CO.
Aktiengesellschaft

CH-4002 Basel(CH)

(72) Erfinder: Angehrn, Peter, Dr.
Bündtenweg 27
CH-4461 Böckten(CH)

(74) Vertreter: Notegen, Eric-André et al,
Grenzacherstrasse 124 Postfach 3255
CH-4002 Basel(CH)

(54) Antibakterielle Komposition.

㉟ Die neue antibakterielle Wirkstoffkomposition, bestehend aus Mecillinam und (2S,3S)-3-[(Z)-2-(2-Amino-4-thiazolyl)-2-[(carboxymethoxy)imino]acetamido]-2-[(carbamoyloxy)-methyl]-4-oxo-1-azetidinsulfonsäure (Verbindung A) oder "Pro-drugs" und/oder pharmazeutisch verwendbaren Salzen davon, besitzt einen ausgeprägten Synergnismus gegenüber Enterobacterstämmen, welche gegenüber Mecillinam resistent und gegenüber der Verbindung A nur mässig empfindlich sind.

F. HOFFMANN-LA ROCHE & CO. Aktiengesellschaft, Basel/Schweiz

0151411

RAN 4410/180

# Antibakterielle Komposition

Die vorliegende Erfindung betrifft eine neue antibakteriell wirksame pharmazeutische Komposition, bestehend aus Mecillinam und (2S,3S)-3-[(Z)-2-(2-Amino-4-thiazolyl)-2-[(carboxymethoxy)imino]acetamido]-2-[(carbamoyloxy)-methyl]-4-oxo-1-azetidinsulfonsäure (welche nachstehend als Verbindung A bezeichnet wird) oder "Pro-drugs" und/oder pharmazeutisch verwendbaren Salzen davon. Sie betrifft ferner ein Arzneimittel mit synergistischen Eigenschaften auf der Basis dieser Komposition und die Verwendung der Komposition bei der Prophylaxe und Therapie bakterieller Infektionen.

Die erfindungsgemässen Arzneimittel enthalten als aktiven Bestandteil die Amidino-Penicillansäure Mecillinam, ein "Pro-drug" und/oder ein pharmazeutisch verwendbares Salz davon in Kombination mit Verbindung A, einem "Pro-drug" und/oder einem pharmazeutisch verwendbaren Salz davon sowie therapeutisch inerte, nicht-toxische, pharmazeutische Trägermaterialien und gegebenenfalls pharmazeutische Hilfsstoffe.

Nt/24.10.84

Mecillinam ist eine bekannte Amidino-Penicillansäure mit antibakterieller Wirkung, vgl. z.B. die Britische Patentschrift Nr. 1.293.590. Die Verbindung A ist ebenfalls eine bekannte Verbindung mit antibakterieller Wirkung, siehe z.B. die Europäische Patentpublikation Nr. 93.376. Es ist ferner bekannt, Mecillinam mit anderen antibiotisch wirksamen Verbindungen zwecks gegenseitiger Potenzierung beider Aktivitäten zu kombinieren, siehe Britische Patentschrift Nr. 2.113.997A.

Stämme mit induzierbarer, chromosomal-determinierter ß-Lactamase können bekanntlich sehr schnell Resistenz gegenüber einer grossen Anzahl von ß-Lactamantibiotika entwickeln, einschliesslich neuer Penicilline und Cephalosporine. Die in dieser Beziehung anpassungsfähigsten Genera von Mikroorganismen sind Enterobacter und Pseudomonas. Verbindungen oder Kompositionen von Verbindungen mit einer Aktivität gegen resistente Stämme von Enterobacter und/oder Pseudomonas sind deshalb von ausserordentlich grossem Wert bei der Bekämpfung bakterieller Infektionen durch diese genannten Mikroorganismen.

Im Rahmen der vorliegenden Erfindung wurde festgestellt, dass die bisher als Kombinationspartner für Mecillinam nicht in Betracht gezogene Verbindung A sich besonders gut für eine derartige Kombination eignet. Die erfindungsgemässe neue Wirkstoffkomposition weist gegenüber bereits bekannten Wirkstoffkompositionen gleicher Wirkungsrichtung therapeutische Vorteile auf, die für den Fachmann nicht vorhersehbar waren. So wurde festgestellt, dass die erfindungsgemässe Wirkstoffkomposition einen ausgeprägten Synergismus gegenüber Enterobacterstämmen besitzt, welche gegenüber Mecillinam resistent und gegenüber der Verbindung A nur mässig empfindlich sind. Wegen des ausgeprägten Synergismus der erfindungsgemässen neuen Wirkstoffkomposition sind jedoch diese Stämme gegenüber dieser Komposition sehr empfindlich.

0151411

In der folgenden Tabelle sind die in in vitro-Vergleichsversuchen ermittelten minimalen Hemmkonzentrationen (MHK-Werte in µg/ml) bei verschiedenen Infektionen mit pathogenen Mikroorganismen für die erfindungsgemässe Komposition dargestellt.

Tabelle 1   In Vitro-Wirkung von Verbindung A   und Mecillinam (MEC) gegenüber verschiedenen Bakterienstämmen   (MHK µg/ml)   Methode: Agarverdünnungstest mit DST Agar (Oxoid)

| | Verbin-dung A | MEC | Verbindung A + MEC | | * | |
| --- | --- | --- | --- | --- | --- | --- |
| | | | 1 + 1 | 1 + 4 | | |
| Enterobacter cloacae MK 373 | 4 | >256 | 1 + 1 | 0.25+ 1 | S | S |
| P99 | 16 | >256 | 2 + 2 | 2 + 8 | S | S |
| 908 | 32 | >256 | 4 + 4 | 2 + 8 | S | S |
| 5 | 8 | >256 | 2 + 2 | 2 + 8 | S | S |
| MK 1617 | 32 | >256 | 4 + 4 | 4 + 16 | S | S |
| 163 | 32 | >256 | 4 + 4 | 2 + 8 | S | S |
| Enterobacter agglomerans 960 | 16 | >256 | 8 + 8 | 4 + 16 | (S) | S |
| Citrobacter freundii 1982 | 4 | >256 | 4 + 4 | 2 + 8 | – | (S) |
| 1385 b | 8 | >256 | 4 + 4 | 2 + 8 | (S) | S |
| Escherichia coli 5/9 | 2 | >256 | 0.5 + 0.5 | 0.25+ 1 | S | S |
| Acinetobacter anitratus 5I-156 | 16 | >256 | 16 + 16 | 8 + 32 | – | (S) |
| Pseudomonas aeruginosa 1920 E | 4 | >256 | 4 + 4 | 4 + 16 | – | – |
| 1937 E | 2 | >256 | 2 + 2 | 4 + 16 | – | – |
| 143724 R | 8 | >256 | 16 + 16 | 16 + 64 | – | – |
| 5646 | 8 | >256 | 16 + 16 | 16 + 64 | – | – |
| 5748 | 8 | >256 | 16 + 16 | 8 + 32 | – | – |

\*  S  : Synergismus (F.I.C.-Index; $\leqslant$ 0.5)
  (S) : Additive Wirkung (F.I.C.-Index; 0.51 – 1.00)
  –  : Indifferenz (F.I.C.-Index; 1.01 – 4.0)

F.I.C. (fractional inhibitory concentrations) Index:

$$\frac{MHK_{\text{Verbindung A in Kombination}}}{MHK_{\text{Verbindung A allein}}} + \frac{MHK_{\text{Mecillinam in Kombination}}}{MHK_{\text{Mecillinam allein}}}$$

(nach G.B.Elion et al, Journal of Biological Chemistry 208; 477–488 [1954])

0151411

Die erfindungsgemässe Komposition kann - wie bereits oben erwähnt - Mecillinam und die Verbindung A als solche oder in Form von Salzen oder von "Pro-drugs" enthalten. Als geeignete Salze der beiden Wirkstoffe kommen vor allem die Alkali-, Erdalkali- und Ammoniumsalze in Frage. Zur Bildung von Ammoniumsalzen kommen Basen, wie Ammoniak, Triäthylamin, Piperidin, Morpholin, Cyclohexylamin, Mono- und Diäthanolamin, Dibenzyläthylendiamin, Benzyl-ß-phenyl-äthylamin und dergleichen in Frage. Mecillinam bildet auch pharmazeutisch verwendbare Salze mit den üblicherweise für diesen Zweck in Frage kommenden anorganischen und organischen Säuren, wie Salzsäure, Schwefelsäure, Phosphorsäure, Ameisensäure, Essigsäure, Bernsteinsäure, Milchsäure, Zitronensäure, Maleinsäure, Fumarsäure, Weinsäure, Methansulfonsäure, p-Toluolsulfonsäure und dergleichen.

Der in dieser Beschreibung verwendete Ausdruck "Pro--drug" umfasst jedes Derivat von Mecillinam bzw. der Verbindung A, welches nach Verabreichung im Körper in Mecillinam bzw. in die Verbindung A übergeht. Beispiele solcher "Pro--drugs" sind im Körper hydrolysierbare Ester, wie niedere Alkanoyloxy-niedere alkyl- und niedere Alkoxycarbonyloxy--niedere alkylester der Carbonsäuregruppen von Mecillinam bzw. der Verbindung A. Diese Ester können wiederum als solche oder als pharmazeutisch verwendbare Salze eingesetzt werden. Als Beispiele solcher Ester seien die Pivaloyloxymethyl- und die Methoxycarbonyloxymethylester erwähnt.

Die mit "nieder" bezeichneten Reste besitzen 1-7, vorzugsweise 1-4 Kohlenstoffatome. Der Ausdruck "Alkyl" bezeichnet geradkettige oder verzweigte, gesättigte Kohlenwasserstoffreste, wie Methyl, Aethyl und t-Butyl. Der Ausdruck "Alkoxy" bezeichnet über ein Sauerstoffatom gebundene Alkylgruppen.

Das Gewichtsverhältnis von Mecillinam zur Verbindung A kann in den erfindungsgemässen Kompositionen und Arzneimit-

- 6 -                    **0151411**

teln im Bereich von 1 zu 100 bis 100 zu 1 variieren und liegt vorzugsweise innerhalb eines Bereichs von 1 zu 10 bis 10 zu 1.

Die Herstellung der Arzneimittel kann in jedem Fachmann geläufiger Weise durch Vermischen der beiden Wirkstoffe mit geeigneten, nicht toxischen, inerten, therapeutisch verträglichen festen oder flüssigen Trägermaterialien einschliesslich der üblichen Hilfsmittel, wie Stabilisierungs-, Konservierungs-, Netz- oder Emulgiermitteln, Mitteln zur geschmacklichen Verbesserung, Salzen zur Veränderung des osmotischen Druckes oder Puffersubstanzen, erfolgen.

Die Applikation kann parenteral oder oral erfolgen. In der Humanmedizin liegt die Tagesdosis zwischen etwa 100 mg und etwa 5 g der Komposition. Zweckmässigerweise wird die Tagesdosis in mehreren Dosierungseinheiten verabreicht, z.B. 2 bis 5 mal täglich je 50-1000 mg der Wirkstoffkomposition. Die in der Humanmedizin zur Anwendung gelangenden Arzneimittel enthalten demnach zweckmässigerweise 50 bis 1000 mg der Wirkstoffkomposition.

Die folgenden Beispiele veranschaulichen die Herstellung von geeigneten Darreichungsformen.

### Beispiel 1

Es werden in an sich bekannter Weise Ampullenfläschchen hergestellt, enthaltend pro Ampullenfläschchen:

| | |
|---|---|
| Mecillinam | 500 mg |
| Mononatriumsalz der Verbindung A | 500 mg |
| Total | 1000 mg |

Die beiden Komponenten werden durch ein 0,3 mm-Sieb gesiebt und gut miteinander vermischt. Der erhaltene Puder wird in 20 ml-Ampullenfläschchen abgefüllt und verschlossen.

Zum Bereitstellen einer Injektionslösung wird der Inhalt vor Gebrauch in 8 bis 10 ml Wasser gelöst.

### Beispiel 2

Es werden in an sich bekannter Weise Tabletten herge-stellt, enthaltend pro Tablette:

| | | |
|---|---:|---|
| Pivmecillinam-hydrochlorid | 100 | mg |
| Verbindung A | 400 | mg |
| 1-Vinyl-2-pyrrolidinon-Polymer | 3 | mg |
| Isopropanol | 0,05 | ml |
| Magnesiumstearat | 5 | mg |

Das Pivmecillinam-hydrochlorid (Pivaloyloxymethylester von Mecillinam-hydrochlorid) wird mit einer Lösung des 1-Vinyl-2-pyrrolidinon-Polymeren in Isopropanol granuliert. Das Granulat wird bei 40°C getrocknet, durch ein 0,7 mm-Sieb gesiebt und mit der Verbindung A und dem Magnesiumstearat gut vermischt. Das Gemisch wird auf einer Tablettierungsmaschine zu Tabletten von 12 mm Durchmesser und einem Gewicht von 560 mg verpresst.

### Beispiel 3

Es werden in an sich bekannter Weise Kapseln herge-stellt, enthaltend pro Kapsel:

| | | |
|---|---:|---|
| Mecillinam | 200 | mg |
| Verbindung A | 200 | mg |
| Magnesiumstearat | 4 | mg |
| Total | 404 | mg |

Die Substanzen werden durch ein 0,6 mm-Sieb gesiebt und gut miteinander vermischt. Das erhaltene Pulvergemisch wird in Gelatine-Kapseln Nr. 0 abgefüllt.

## Beispiel 4

Es werden in an sich bekannter Weise Präparate zur intramammalen Injektion hergestellt, enthaltend pro Injektionsspritze:

| | |
|---|---:|
| Mecillinam | 150 mg |
| Mononatriumsalz der Verbindung A | 150 mg |
| Aluminiummonostearat | 100 mg |
| 12-Hydroxystearin | 100 mg |
| flüssiges Paraffin | <u>4500 mg</u> |
| Total | 5000 mg |

Das Aluminiummonostearat und das 12-Hydroxystearin werden im flüssigen Paraffin bei 130°C gelöst und auf 30°C abgekühlt. Das Mecillinam und das Mononatriumsalz der Verbindung A werden zu Partikeln unter 50 Mikron gemahlen und danach in der intramammalen Grundlage dispergiert. Nach Homogenisierung unter Verwendung einer Kolloidmühle wird das intramammale Präparat in Plastik-Injektionsspritzen abgefüllt.

## Beispiel 5

Es werden in an sich bekannter Weise Sachets folgender Zusammensetzung hergestellt:

| | |
|---|---:|
| Mecillinam | 3000 g |
| Mononatriumsalz der Verbindung A | 1500 g |
| Sucrose | 30000 g |
| Natriumcitrat | 250 g |
| Natriumcarboxymethylcellulose | 250 g |
| Pfefferminzaroma | 10 g |

Die Komponenten werden durch ein Sieb von 0,5 mm gesiebt, gemischt und in Sachets enthaltend 3,5 g des Gemisches gefüllt. Der Inhalt eines Sachets wird vor Gebrauch in ungefähr 10 ml Wasser gelöst.

0151411

## Patentansprüche

1. Komposition bestehend aus Mecillinam und (2S,3S)-3-
-[(Z)-2-(2-Amino-4-thiazolyl) -2-[(carboxymethoxy)imino]-
acetamido] -2-[(carbamoyloxy)methyl]-4-oxo-1-azetidinsulfon-
säure oder "Pro-drugs" und/oder pharmazeutisch verwendbaren
Salzen davon.

2. Komposition gemäss Anspruch 1, gekennzeichnet durch
ein Gewichtsverhältnis der beiden Bestandteile von 1 zu 100
bis 100 zu 1.

3. Komposition gemäss Anspruch 2, gekennzeichnet durch
ein Gewichtsverhältnis der beiden Bestandteile von 1 zu 10
bis 10 zu 1.

4. Arzneimittel enthaltend eine Komposition gemäss einem
der Ansprüche 1-3 und ein therapeutisch inertes Trägermaterial.

5. Arzneimittel gemäss Anspruch 4, enthaltend 50-1000 mg
der Komposition.

6. Verwendung einer Komposition gemäss einem der Ansprüche 1-3 bei der Bekämpfung bzw. Verhütung bakterieller
Infektionen.

\*\*\*

**Europäisches Patentamt**

**EUROPÄISCHER RECHERCHENBERICHT**

**0151411**
Nummer der Anmeldung

EP  85 10 0428

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP-A-0 050 965  (TAKEDA) <br> * Ansprüche 1-5 * | 1 | A 61 K  31/43 // <br> (A 61 K  31/43 <br> A 61 K  31:425) |
| D,Y | GB-A-2 113 997  (LEO PHARM. PRODUCTS LTD.) <br> * Insgesamt * | 1-6 | |

RECHERCHIERTE SACHGEBIETE (Int. Cl.4)

A 61 K  31/00
C 07 D 499/00
C 07 D 417/00
C 07 D 205/00

Der vorliegende Recherchenbericht wurde für alle Patentansprüche erstellt.

| Recherchenort <br> DEN HAAG | Abschlußdatum der Recherche <br> 24-04-1985 | Prüfer <br> CHOULY J. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82